# EUROPEAN PATENT SPECIFICATION

(11) **EP 4 683 689 B1**
(45) Date of publication and mention of the grant of the patent: **19.08.2026**
(21) Application number: 23724116.1
(22) Date of filing: 20.04.2023
(51) Int. Cl.: A61M 5/142, A61M 5/36

(54) **AIR IN LINE MEASUREMENT SYSTEM FOR INFUSION PUMPS**
LUFT-IN-LINE-MESSSYSTEM FÜR INFUSIONSPUMPEN
SYSTÈME DE MESURE DE L'AIR EN LIGNE POUR POMPES À PERFUSION

(43) Date of publication of application: 28.01.2026
(73) Proprietor: CareFusion 303, Inc., San Diego, CA 92130 (US)
(72) Inventor: CAI, Frank, San Diego, California 92130 (US)
(74) Representative: Zenz Patentanwälte Partnerschaft mbB
(86) International application number: PCT/US2023/019296
(87) International publication number: WO 2024/220079

(56) References cited:
- EP-A1- 3 988 142
- US-A- 5 123 275
- US-A- 6 142 008
- US-B2- 8 910 370

## Description

### BACKGROUND

Infusion pumps are complex electromechanical devices used to deliver fluids into a patient's body in a controlled manner. They typically serve the needs of hospital-bound patients, where life-saving medication is normally delivered via intravenous infusion. Thus, reliability of infusion pumps and patient safety are extremely important.

Reliability of infusion pumps and patient safety can be negatively affected when air enters the fluid line during an infusion. Thus, infusion pumps typically include an air-in-line detector that may be used for detection of air in the fluid line, which is important for patient safety because small volumes of air injected intravenously are considered a hazard. If excessive air is detected in the fluid line, the infusion pump stops the infusion and generates an alarm to alert the caregiver.

Air-in-line sensor assemblies typically include a sender and a receiver, which reads small voltages when compatible fluid tubing is used and there is no air at the sensor. The sender and receiver typically have a fixed distance between them, which means manufacturing tolerances, tubing placement, and collapsing of the tubing due to occlusions can have the undesired effect of stopping an infusion and triggering "nuisance" alarms (e.g., alarms associated with a particular condition but raised due to an alternate or unrelated condition). Raising alarms can consume valuable resources of an infusion device. For example, some infusion devices may include visual or audio alert devices that can be activated when certain conditions are detected. Activation of these devices can consume valuable resources (e.g., power, processing time, network traffic, network interface, etc.) of the infusion device that might otherwise be used to deliver fluids or other operations. Some alarms may interrupt operation of the infusion device. The process of interrupting, confirming a restart, and restarting the infusion device can also consume resources. For example, there may be momentum built up in a cam used to drive the pump that, if stopped, would be lost. In losing the momentum, the pump may need extra cycles or other resources to return to the pre-pause operational efficiency. This introduces potential loss due to the alarm. Stopping and restarting an infusion may also impact characteristics of the flow because the tubing or other pumping elements may relax or change temperature after a pause. Nuisance alarms can prevent patients from receiving medication, and they can take away time from a caregiver that could have been used to treat other patients.

US 8 910 370 B2 discloses a universal air bubble detector allowing for use with a variety of sizes and types of tubing. The detector maintains proper alignment of a sensor emitter and receiver with different sizes of tubing. The detector may be mounted on existing equipment or may be used to monitor a tubing at any position along the tubing, and may operate in a stand alone mode or in combination with existing equipment.

US 6 142 008 A discloses a system and a method for detecting the presence of air bubbles in an intravenous (IV) line supplying a medicinal liquid to a patient. An air bubble sensor includes an ultrasonic transmitter acoustically coupled to an ultrasonic receiver to detect the presence of a gas (e.g., air) in a portion of a tube comprising the IV line. The transmitter and receiver are mounted on pivoting transducers that are disposed on opposite sides of the tube. A spring biases the transducers inwardly toward each other so that the transmitter and receiver contact opposite sides of the tubing. This assembly automatically accommodates different sizes of tubing and tubing of a relatively wide range of stiffness. The tube is connected to a disposable pumping cassette that is engaged in a pump chassis on which the transducers are pivotally mounted. A user actuated plunger on the pump chassis is depressed to cause the transmitter and receiver to move away from the tube when the pumping cassette is removed from or inserted into the interior of the pump chassis. A controller precisely monitors the flow of medicinal liquid through the tubing to detect the size of gas bubbles and total volume of gas infused. The controller automatically compensates for minor contamination of the exterior surface of the tube, e.g., if the surface is wet with a liquid.

EP 3 988 142 A1 discloses a bubble detector for detecting existence of bubbles in a liquid flowing through a tube by bringing a first ultrasonic element disposed on a first support member (sensor block) and a second ultrasonic element disposed on a second support member (cover) into tight contact with a tube. A pair of side blocks are disposed in a direction intersecting an arrangement direction of ultrasonic elements. The distance between the ultrasonic elements is adjusted simultaneously with adjustment of the distance between the paired side blocks in accordance with the diameter of the tube so that the tube is rectangularly pressed. This prevents a flow rate reduction caused by the elliptically pressed tube.

US 5 123 275 A discloses a sensor system having a two-housing arrangement, each housing of which contains a transducer. One housing includes the transmitter transducer and a flat engaging surface for pressing against the fluid line and deforming it. The other housing includes the receiver transducer mounted so that it is coupled to the bottom of a rectangular U-shaped channel for receiving the fluid line. A spring biases the flat surface of the first housing into contact with the fluid line and deforms the fluid line into the shape of the channel so that the fluid line fills the channel. The channel is shaped to force the fluid line into a general rectangular shape as it is pressed into the channel by the flat surface. Two walls of the deformed fluid line are oriented in a direction perpendicular to the flat engaging surface of the first housing and provide an opposing force to the flat surface. The fluid line stiffness and pressure of the fluid within act as an opposing spring to limit the travel of the transmitter transducer housing to result in firm and continuous contact between the transducers and the fluid line. A stop mechanism prevents the two housings from contacting.

### SUMMARY

The invention is defined in the independent claims. Preferable embodiments are further laid out in the dependent claims. Based on the discussion above as well as other problems and disadvantages of the related art, there is a need for improved air-in-line detection systems and methods that allow infusion pumps to differentiate between actual air in the system and an upstream occlusion, while compensating for manufacturing tolerances of the tubing or pump, thereby increasing device performance and patient safety, conserving valuable resources, and decreasing potential loss and harmful impacts to the characteristics of the flow of therapeutic fluids into the patient. The improved air-in-line detection systems and methods described herein achieve these advantages by providing a constant force between the sender and receiver of the air-in-line sensor onto the fluid tubing, and in some instances, adjusting alarm thresholds according to baseline tubing thickness determinations.

According to various aspects of the subject technology, an infusion pump comprises a housing including a tubing receiving area, and an air-in-line sensor including a first element coupled to the housing behind the tubing receiving area, and a second element configured to be positioned over the tubing receiving area and biased by a constant force toward the first element. The infusion pump further comprises a position sensor coupled to the housing and configured to detect a position of the second element, and a processor configured to: receive confirmation that a fluid tubing is installed in the tubing receiving area; determine a distance between the first element and the second element based on the position of the second element detected by the position sensor; set a baseline tubing thickness of the fluid tubing according to the distance between the first element and the second element; configure an alarm threshold of the air-in-line sensor according to the baseline tubing thickness; and perform an infusion while operating the air-in-line sensor according to the alarm threshold.

According to various aspects of the subject technology, a method of operating an infusion pump includes, at a processor of the infusion pump: receiving confirmation that a fluid tubing is installed in a tubing receiving area of the infusion pump; determining a distance between a first element and a second element of an air-in-line sensor of the infusion pump using a position sensor coupled to a housing of the infusion pump and configured to detect a position of the second element, wherein: the first element is coupled to the housing behind the tubing receiving area, and the second element is positionable over the tubing receiving area and biased with a constant force toward the first element; setting a baseline tubing thickness of the fluid tubing according to the distance between the first element and the second element; configuring an alarm threshold of the air-in-line sensor according to the baseline tubing thickness; and performing an infusion while operating the air-in-line sensor according to the alarm threshold.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Detailed Description below, in conjunction with the following drawings. Like reference numerals refer to corresponding parts throughout the drawings and description.
FIG. 1 depicts an example infusion pump setup, shown in use in its intended environment.
FIGS. 2A-2B depict perspective views of internal components of an infusion pump, including an air-in-line sensor assembly.
FIG. 2C depicts a detailed view of the air-in-line sensor assembly of the infusion pump.
FIGS. 3A-3B depict example default and adjusted ranges for readings from the air-in-line detector of an infusion pump, based on the distance between the sending and receiving elements of the air-in-line sensor.
FIG. 4 depicts an example process for adjusting air-in-line alarm thresholds when operating an infusion pump.
FIG. 5 is a conceptual diagram illustrating an example electronic system for adjusting air-in-line alarm thresholds when operating an infusion pump.

### DETAILED DESCRIPTION

Reference will now be made to implementations, examples of which are illustrated in the accompanying drawings. In the following description, numerous specific details are set forth, in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

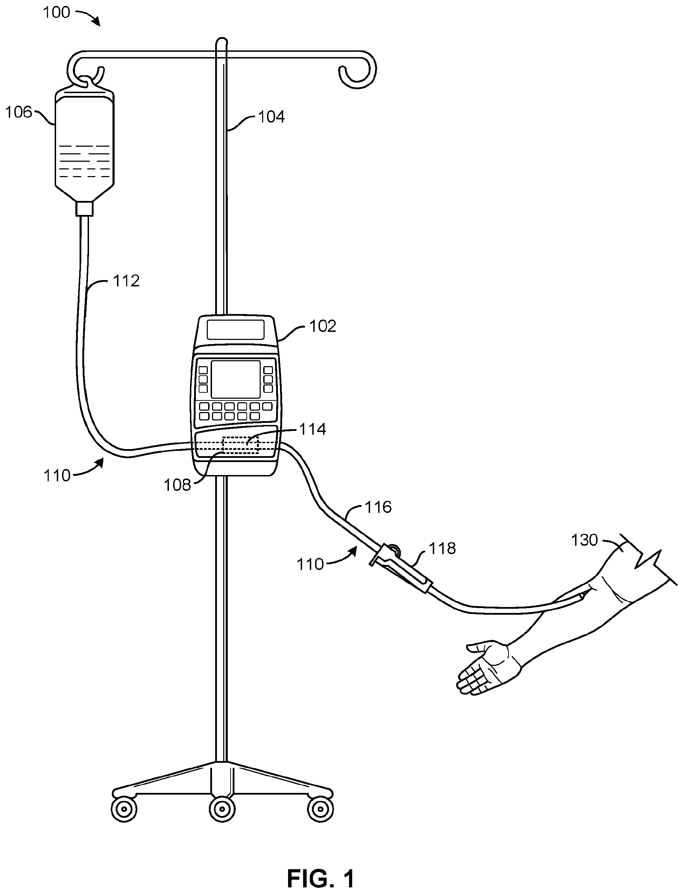

FIG. 1 depicts an example infusion pump 102, shown in use in its intended environment 100, according to various aspects of the subject technology. In particular, the infusion pump 102 is shown mounted to an intravenous (IV) pole 104 on which a fluid supply 106 containing an IV fluid is held. The fluid supply 106 is connected in fluid communication with a flexible pumping fluid line (also referred to as fluid tubing) of an administration set 110. As used herein, the terms "fluid line" and "fluid tubing" may refer to the fluid line within an administration set, or to the administration set itself. The fluid line includes an upstream portion 112, a pump-mounted portion 114, and a downstream portion 116. The fluid line comprises IV tubing typically used in a hospital or medical environment, and is made of any type of flexible tubing appropriate for use in infusing therapeutic fluids into a patient, such as polyvinylchloride (PVC). The pump-mounted portion 114 is mounted in operative engagement with a pumping mechanism 108 (e.g., a peristaltic pump), configured to propel fluid from the upstream portion 112 through the downstream portion 116 to a patient 130 (e.g., to a patient's arm).

The fluid line portions 112, 114, and 116 of the administration set 110 may be a continuous length of flexible tubing, with the portions defined by the location of the pumping mechanism 108. A roller clamp 118, providing for mechanical compression of the fluid line to block the flow of fluid, may be positioned on the downstream portion 116 of the fluid line between the pump 102 and the patient 130. In this context, the term "upstream" refers to the portion of the fluid line that extends between the fluid supply 106 and the pumping mechanism 108, and the term "downstream" refers to the portion of the fluid line that extends from the pumping mechanism 108 to the patient 130.

The pumping mechanism 108 directly acts on portion 114 of the fluid line. Portion 114 of the fluid line connects the upstream portion 112 to the downstream portion 116 to form a continuous fluid conduit extending from the fluid supply 106 to the patient 130. As such, the infusion pump 102 causes fluid in the fluid line to move downstream from the fluid supply 106 to the patient 130. Specifically, the pumping mechanism 108 acts as a flow control device of the infusion pump 102 to move fluid though the fluid line to the patient.

The type of pumping mechanism 108 may vary and may be, for example, a multiple-finger peristaltic pump. For example, the pumping mechanism may include a plurality of occluding fingers and/or pumping fingers configured to apply pressure in sequential locations of the fluid line beginning at the upstream end of the pumping mechanism and working toward the downstream end. At least one finger is always pressing hard enough to occlude the fluid line. As a practical matter, one finger does not retract from occluding the fluid line until the next one in sequence has already occluded the fluid line; thus, at no time is there a direct fluid path from the fluid supply 106 to the patient 130. Each finger is coupled to a cam, and the cams corresponding to the fingers are coupled to a camshaft, which is controlled by a motor. In some implementations, one complete revolution of the camshaft causes a single pumping cycle.

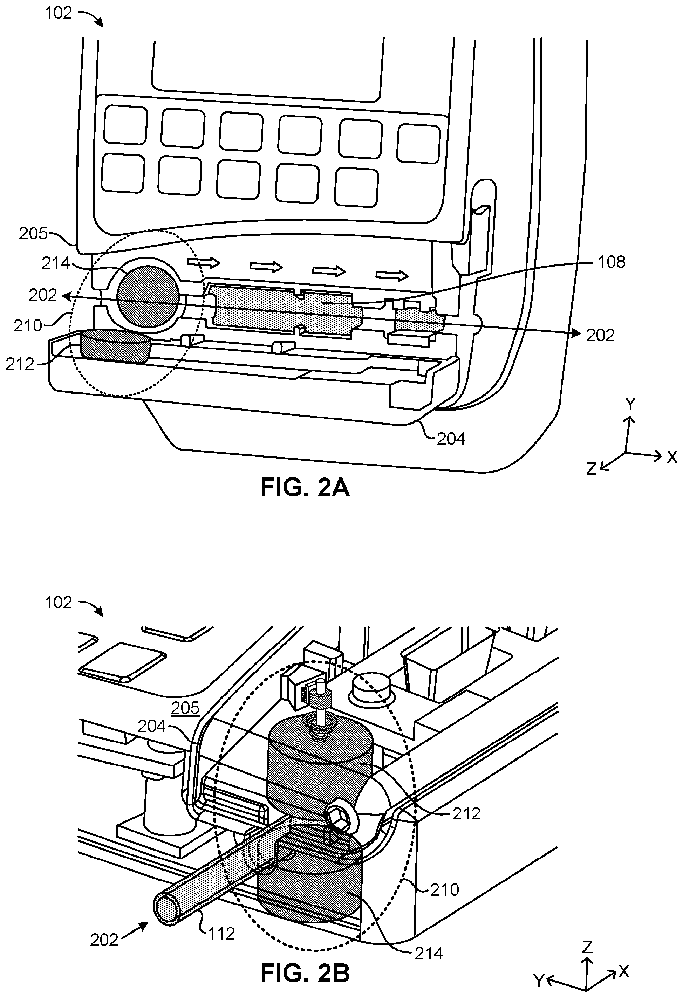

FIGS. 2A-2B depict perspective views of internal components of the infusion pump 102, including an air-in-line sensor assembly 210, according to various aspects of the subject technology. The air-in-line sensor assembly 210 may be used for detection of air in the fluid line, which is important for patient safety because small volumes of air injected intravenously are considered a hazard. If excessive air is detected in the fluid line, the infusion pump 102 stops the infusion and outputs an alarm to alert the caregiver. Further, proper detection of air in the fluid line optimizes operational efficiency of the infusion pump and increases patient safety by conserving valuable resources and decreasing potential loss and harmful impacts to the characteristics of the flow of therapeutic fluids into the patient.

The air-in-line sensor assembly 210 is located in front of and behind a tubing receiving area 202 (also referred to as a tubing channel), which may be accessed by opening a door 204 of the infusion pump 102 (e.g., uncoupling at least a portion of the door 204 from a housing 205 of the pump 102). The tubing receiving area 202 is a path through the interior of the infusion pump 102, through which the fluid tubing (portions 112, 114, 116 depicted in FIG. 1) may be mounted. Upon being mounted into the tubing receiving area 202, the fluid tubing passes through the air-in-line sensor assembly 210 and the pumping mechanism 108. In some implementations, the fluid tubing may also pass through other sensors not shown in the figures (e.g., one or more occlusion sensors and/or flow sensors) before exiting the infusion pump 102.

The air-in-line sensor assembly 210 includes an air-in-line detector (also referred to as an air-in-line sensor) consisting of a pair of elements 212, 214. One of the elements is configured as a transmitter (also referred to as a sending element or a sending sensor) and the other of the elements is configured as a receiver (also referred to as a receiving element or a receiving sensor). In some implementations, element 212 is the transmitter and element 214 is the receiver, and in other implementations, element 212 is the receiver and element 214 is the transmitter.

In some implementations, the transmitter and receiver elements 212, 214 are a pair of ceramic ultrasonic sensors that are used in tandem with a processor implementing software algorithms to detect the presence of air in the fluid line. When an ultrasonic signal traveling between the transmitter and receiver passes through air in the fluid line, the air-in-line detector outputs a voltage signal. The voltage signal is representative of an amount of air detected within the tubing of portion 112 of the fluid line. The air-in-line detector continually monitors the presence of air within the fluid line 112 and translates the measured readings (e.g., air bubble size, or number of air bubbles) to corresponding voltages. These voltages are provided (e.g., by analog-digital converters within the pump) to a processor. The processor compares the voltage value readings to predetermined thresholds (e.g., an air bubble size threshold, or a cumulative air bubble count). In some implementations, the processor compares the voltage readings to the predetermined thresholds for a moving window of time. Satisfaction of a threshold may include, for example, an average or mean of the voltage values measured during the window of time satisfying (e.g., meeting and/or exceeding) a threshold value, or a minimum or a maximum value within that window satisfying the threshold value.

An alarm may occur when the air-in-line detector detects either (i) a series of smaller air bubbles that correspond to a dangerous total sum of air, or (ii) a continuous/single air bubble that is of an unacceptable volume. The acceptable air volume thresholds may be configured to balance the tradeoffs between patient safety and nuisance alarms. If the air-in-line detector is too sensitive, then nuisance air-in-line alarms may occur due to misdetection of air in the fluid tubing, which can lead to complaints. Conversely, if the air-in-line detector is too insensitive, unacceptable volumes of air may not be detected, which can cause patient harm.

The sensing elements of the air-in-line detector are mounted to the infusion pump 102 so that they are on opposite sides of the fluid tubing 112 during an infusion. Specifically, in some implementations, element 212 may be mounted to the door (in front of the tubing receiving area 202), and element 214 may be mounted to the body of the infusion pump (behind the tubing receiving area 202). As such, when the fluid tubing 112 is mounted in the tubing receiving area 202 and the door 204 is closed (e.g., coupled to a housing 205 of the pump as shown in FIG. 2B), sensing elements 212 and 214 are positioned at opposite sides of the fluid tubing 112, with element 212 positioned in front of the fluid tubing and element 214 positioned behind the fluid tubing.

In some implementations, both sensor elements 212 and 214 may be coupled to the infusion pump without either element being mounted to the door. In such implementations, the fluid tubing 112 may be threaded between the sensor elements 212 and 214. Element 212 may be movable into a position over the fluid tubing 112 as part of the loading process of the administration set. For ease of explanation and to avoid obscuring more pertinent aspects of this disclosure, the following discussion describes implementations in which sensor element 212 is mounted to the door. However, the concepts described herein apply equally to any implementation in which one air-in-line sensor element is movable with respect to the other, regardless of how the sensor elements are coupled to the infusion pump (e.g., with or without door mounting).

If the air-in-line sensor elements 212, 214 are maintained at a fixed distance from each other, as in conventional infusion pumps, different tubing sizes may affect the sensitivity of the air-in-line detector. For instance, a fluid tube having a smaller diameter would cause more air to be detected between air-in-line sensor elements positioned a fixed distance apart from each other. If there is more air between the air-in-line sensor elements, then the air-in-line detector may be more sensitive to additional air detected in the fluid tubing, and a quantity of air passing through the tubing that would not have otherwise raised an alarm could cause a nuisance alarm.

Rather than having a fixed distance between sensor elements, implementations of the air-in-line detector as described herein include sensor elements separated from each other at a variable distance. Specifically, at least one of the air-in-line sensor elements 212, 214 is configured to be movable with respect to the other. At least one sensor element (e.g., 212) is configured to apply a constant force to the tubing 112 in the direction of the other sensor element (e.g., 214). This constant force causes the air-in-line sensor elements to grip the tubing, regardless of the diameter of the tubing. This enhanced grip provides a much more consistent signal, regardless of the tubing size.

While FIG. 2B depicts sensor element 212 applying a force on the tubing 112 while element 214 is fixedly coupled to the infusion pump 102, some implementations may configure sensor element 214 to apply the force on the tubing 112 while sensor element 212 is fixedly coupled to the door 204. Yet other implementations may configure both sensor elements 212, 214 to apply force on the tubing 112. In any case, the force applied on the tubing 112 by the sensor element(s) causes the sensor elements to have a variable distance between them based on variable tubing sizes.

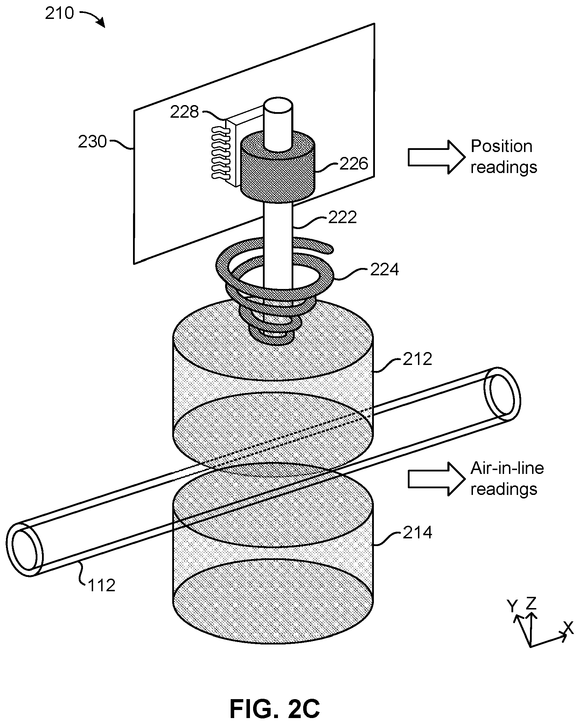

FIG. 2C depicts a detailed view of the air-in-line sensor assembly 210, according to various aspects of the subject technology. In addition to sending and receiving sensor elements 212 and 214, the air-in-line sensor assembly 210 includes a rod 222 on which a biasing element, such as a spring 224, and a magnet 226 are mounted, and a position sensor assembly 230 including a position sensor 228. In some implementations, the spring 224 is coupled to the rod 222 with a spring seat, and the rod 222 has a bearing to hold it in place in the door 204. Thus, rod 222 may be referred to as a spring-biased or spring-loaded rod 222.

The spring 224 may be a compact spring that sits between the door 204 and the sensor element 212, configured to push the sensor element 212 against the tubing 112. The spring 224 causes the sensor element 212 to apply a constant spring force to the tubing 112. The spring force may be characterized by a spring rate, also known as a spring constant, and it may be measured by the pounds of force required to cause the spring to travel one inch of compressed distance. In general, the spring force of the spring 224 is selected to be high enough to apply pressure on the tubing, but low enough so as to not collapse the tubing or restrict the flow of fluid to the patient. In some implementations, the spring force of the spring 224 is selected from a range of 2-3 pounds. The constant force of the sensor element 212 on the tubing 112 caused by the spring force of the spring 224 makes the voltage signal corresponding to air-in-line readings more consistent, since the amount of air between the air-in-line sensor elements 212, 214 does not change with the diameter of the tubing.

The position sensor assembly 230, on which position sensor 228 is mounted, is coupled to the body of the infusion pump 102. Since the magnet 226 is coupled to the rod 222 that moves with the sensor element 212 and door 204, and the position sensor 226 is fixed to the body of the pump 102, the magnet moves up and down the Z-axis (or forwards and backwards, depending on the frame of reference) with respect to the position sensor 226. Based on the movement of the magnet 226 with respect to the position sensor 226, the position sensor 226 can determine how far the spring 224 has compressed. Specifically, the position sensor 226 determines a position of the rod-mounted magnet 226 with respect to the position sensor 228, and since the magnet 226 is coupled to the spring 224, changes in the position of the magnet 226 correspond to changes in the position of the spring 224, which correspond to changes in the position of the sensor element 212.

As a result of the variable distance between air-in-line sensor elements 212, 214, the infusion pump 102 is more tolerant to different sizes of tubing 112, thus providing more consistent and higher quality air-in-line detection signals when using tubing that would not have otherwise been able to facilitate the transmission of ultrasonic signals between the air-in-line sensor elements 212, 214.

In some implementations, a processor of the infusion pump 102 uses the position of the sensor element 212 to tune the air-in-line voltage readings. The processor may determine an initial thickness of the tubing 112 based on the position of the sensor element 212 with respect to the position of the other sensor element 214. Stated another way, by determining the position of the sensor element 212, the processor may determine the distance between sensor elements 212 and 214 and set the tubing thickness based on the determined distance. The processor may set a baseline pressure reading based on the initial tubing thickness (at the beginning of an infusion). The processor may use the baseline pressure reading to set a range of voltage readings corresponding to air-in-line detections. Thus, a higher quality signal may be obtained from the air-in-line sensors.

In some implementations, rather than (or in addition to) determining the position of the air-in-line sensor element 212 based on readings from a position sensor, other sensors may be used to detect the stiffness and/or preload of the spring 224 to determine how far the air-in-line sensor element 212 has been moved, and thus, determine the position of the air-in-line sensor element 212.

In some implementations, rather than (or in addition to) a variable-position door-mounted element 212 being configured to move with respect to a fixed pump body-mounted element 214, the body-mounted element 214 may be configured to move with respect to a fixed door-mounted element 212 (or with respect to a variable-position door-mounted element 212). To accomplish this, a rod, spring, and magnet may be coupled to the body-mounted element 214 in a manner similar to that described above with reference to rod 222, spring 224, and magnet 226 in FIG. 2C, but with the position sensor assembly 230 (including position sensor 228) being coupled to the pump body and positioned near the magnet mounted to element 214.

In some implementations, a processor of the infusion pump 102 may discern between air-in-line hazards and upstream occlusions using the air-in-line sensor assembly 210.

When an occlusion occurs in the upstream portion 112 of the fluid tubing, the tubing fills with air due to negative pressure created by the pumping mechanism 108, and as a result, the tubing collapses. As the tubing collapses, it pulls away from the air-in-line sensing elements 212, 214, causing the air-in-line detector to output a 0mV reading due to the air-filled tubing no longer transmitting ultrasonic signals between the elements 212, 214.

When an air-in-line hazard occurs in the pump-mounted portion 114 of the fluid tubing, the tubing itself does not collapse. Rather, the tubing maintains contact with the sensor elements 212, 214 while the air bubbles pass through, causing only short periods of voltage drops due to the air bubbles causing the fluid to less efficiently transmit ultrasonic signals between the sensor elements 212, 214.

Due to the difference in output of the air-in-line detector for occlusions versus air-in-line hazards, the processor can determine whether an air-in-line alarm corresponds to (i) an upstream occlusion (e.g., by determining that the output voltage has dropped below an occlusion threshold for a given amount of time), or (ii) an air-in-line hazard (e.g., by determining that the output voltage has not dropped below an occlusion threshold for a given amount of time). Thus, when an alarm sounds, the clinician can diagnose the issue more efficiently and resume the infusion sooner.

While the implementations described above use a spring-biased or spring-loaded rod to provide a constant force that biases element 212 toward element 214, other implementations may provide a constant force without the use of a spring-loaded rod. In other words, a spring is just one example of a biasing element that may be configured to bias one air-in-line sensor element toward the other air-in-line sensor element. For example, a hinge, an elastic material, a dynamically compressible material, or any other mechanical alternative to a spring may be used to apply a constant force that biases element 212 toward element 214 (or that biases element 214 toward element 212). For ease of explanation and to avoid obscuring more pertinent aspects of this disclosure, the following discussion is with respect to implementations in which a spring-loaded rod provides a constant force on one air-in-line sensor toward the other air-in-line sensor. However, the concepts described herein apply equally to any implementation in which any other mechanical component applies a constant force on one air-in-line sensor toward the other air-in-line sensor.

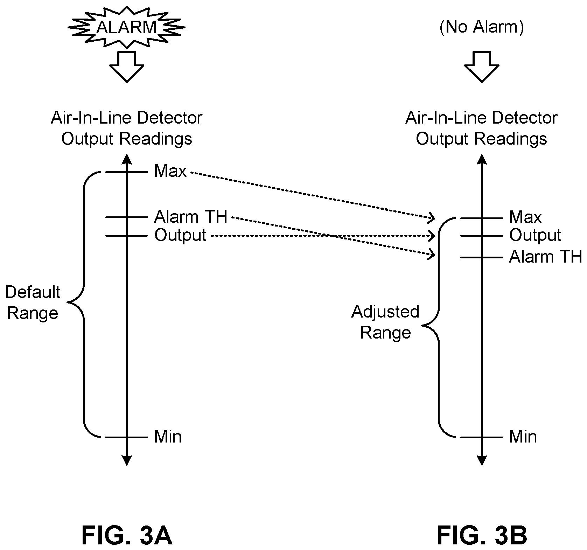

FIGS. 3A-3B depict example default and adjusted ranges for readings from the air-in-line detector of an infusion pump, based on the distance between the sending and receiving elements of the air-in-line sensor.

In FIG. 3A, a default range is set according to a predetermined specification for the air-in-line detector. The maximum output value (Max) corresponds to fluid-filled tubing that transmits ultrasonic signals between sensor elements 212, 214 efficiently due to no air being present in the tubing. The minimum output value (Min) corresponds to air-filled tubing that does not transmit ultrasonic signals between sensor elements 212, 214. An alarm threshold (Alarm TH) may be set with reference to the maximum reading (e.g., an average of 80% of the maximum reading over a given window of time). In this scenario, if the air-in-line detector outputs a voltage (Output) below the alarm threshold for a given window of time, the infusion pump may pause the infusion and outputs an alarm to notify the caregiver.

In FIG. 3B, the processor has determined that the distance between elements 212, 214 is larger than the default distance in FIG. 3A, and thus, the tubing has a larger diameter. The processor tunes the range of air-in-line detector outputs based on the lager tubing size. The minimum output value (Min) remains the same, as this value still corresponds to air-filled tubing that does not transmit ultrasonic signals between sensor elements 212, 214. However, the maximum output value (Max) is adjusted to the value of the air-in-line detector output at the beginning of the infusion (or during a diagnostic prior to beginning the infusion). The new maximum output value corresponds to fluid-filled tubing (having a lager size than that in FIG. 3A) that transmits ultrasonic signals between sensor elements 212, 214 efficiently due to no air being present in the tubing.

The reason the adjusted maximum output value has decreased is the increased tubing size causes the sensor elements 212, 214 to be slightly farther apart than in FIG. 3A, and there is more fluid between them. Thus, the ultrasonic signals are transmitted between the sensor elements 212, 214 with slightly less efficiency, thereby causing the output voltage of the air-in-line detector to slightly decrease.

Referring back to FIG. 3B, the alarm threshold (Alarm TH) is adjusted according to the new maximum output reading (e.g., an average of 80% of the maximum reading over a given window of time). In this scenario, the same output value (Output) as that in FIG. 3A does not trigger an alarm because the output value is no longer below the alarm threshold. The decreased alarm threshold is a better threshold for the increased tubing size, as it causes fewer nuisance alarms.

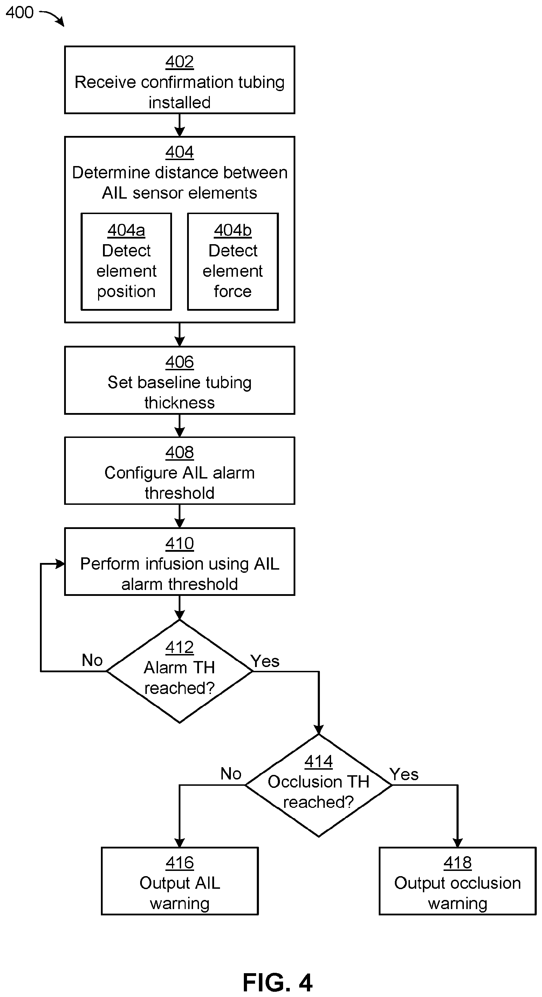

FIG. 4 depicts an example process 400 for adjusting air-in-line alarm thresholds, according to aspects of the subject technology. For explanatory purposes, the various blocks of example process 400 are described herein with reference to FIGS. 1-3B, and the components and/or processes described herein. The one or more of the blocks of process 400 may be implemented, for example, by one or more computing devices including, for example, pump 102. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 400 are described as occurring in serial, or linearly. However, multiple blocks of example process 400 may occur in parallel. In addition, the blocks of example process 400 need not be performed in the order shown and/or one or more of the blocks of example process 400 need not be performed.

Prior to infusing the patient, an administration set 110 is loaded with fluid from a fluid supply 106 and installed in the pump. A processor of the pump receives confirmation (402) that the administration set (fluid tubing) is properly installed in a tubing receiving area 202 of the pump and a door 204 is secured to a housing 205 of the pump (the door is closed). At this point, the primed administration set is connected to a fluid supply (e.g., supply 106 via portion 112) and is installed in the pump (e.g., portion 114), but may not yet be connected to a patient. Instead, the downstream portion of the administration set (e.g., 116) may be temporarily inserted into a container for collecting excess fluid.

Prior to beginning the infusion, the processor determines (404) a distance between first and second sensing elements (212, 214) of an air-in-line sensor of the pump based on the position of the second element detected by a position sensor 228. In some implementations, a magnet 226 is coupled to a spring-loaded rod 222 that is coupled to the second element 212, and the position sensor is configured to detect (404a) the position of the second element based on a position of the magnet. In some implementations, a force sensor coupled to the spring-loaded rod, and the force sensor is configured to detect (404b) the position of the second element based on a stiffness of the spring 224.

In some implementations, the force applied by the spring-loaded rod on the second element causes the second element to apply a constant force on the fluid tubing while preventing substantial obstruction of the fluid tubing. In some implementations, the force applied by the spring-loaded rod on the second element is a constant force selected from a range of 2-3 pounds per inch.

The processor proceeds to set (406) a baseline tubing thickness of the fluid tubing according to the distance between the first element and the second element. In some implementations, the baseline tubing thickness corresponds to the distance between the first element and the second element while the fluid tubing is installed in the tubing receiving area and the door is secured to the housing. In some implementations, the processor is configured to tune a range of readings of the air-in-line sensor using the baseline tubing thickness and configure an air-in-line alarm threshold based on the tuned range of readings. In some implementations, the alarm threshold is configured based on a predetermined percentage of a maximum or minimum output reading of the tuned range of readings of the air-in-line sensor (e.g., as described above with reference to FIGS. 3A-3B).

The processor configures (408) an alarm threshold of the air-in-line sensor according to the baseline tubing thickness (e.g., as described above with reference to FIGS. 3A-3B). The infusion pump then continues with normal startup procedures (e.g., the clinician enters a flow rate and volume to be infused (VTBI) for the infusion being programmed). As part of these startup procedures, the clinician attaches the downstream portion of the administration set 116 to the patient and proceeds with the infusion. As such, the processor causes the infusion pump to perform (410) an infusion while operating the air-in-line sensor according to the configured alarm threshold.

While performing the infusion, the processor determines (412) whether the alarm threshold has been reached. If not, the system continues performing the infusion. If so, in response to determining that the alarm threshold has been reached, the processor compares an output of the air-in-line sensor to an occlusion threshold and determines (414) whether the output reaches an occlusion threshold. The processor outputs (416) an air-in-line alarm when the output of the air-in-line sensor is greater than the occlusion threshold, and outputs (418) an occlusion alarm when the output of the air-in-line sensor is less than or equal to the occlusion threshold.

By improving air-in-line detection of an infusion pump, the above-described example process 400 and related features and applications optimize performance of the infusion pump, thereby increasing patient safety, conserving valuable resources, and decreasing potential loss and harmful impacts to the characteristics of the flow of therapeutic fluids into the patient.

Many of the above-described example process 400 and related features and applications may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

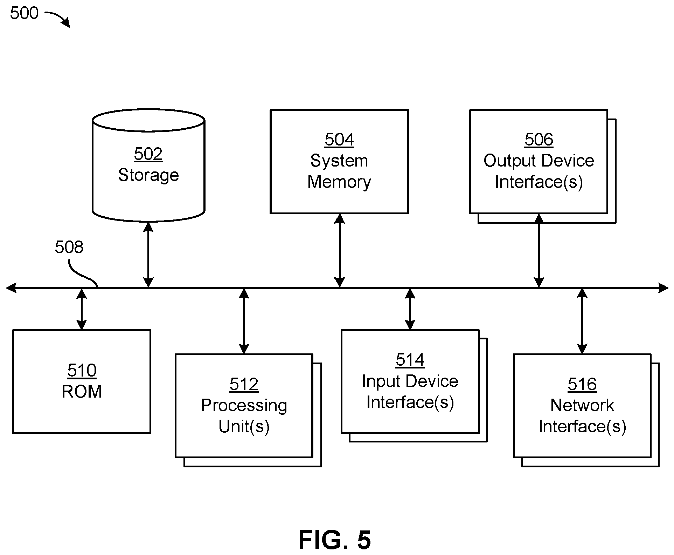

FIG. 5 is a conceptual diagram illustrating an example electronic system 500 for adjusting air-in-line alarm thresholds when operating an infusion pump, according to aspects of the subject technology. Electronic system 500 may be a computing device for execution of software associated with one or more portions or steps of method 400, or components and methods provided by FIGS. 1-4, including but not limited to computing hardware within infusion pump 102 and/or any computing devices or associated terminals disclosed herein. In this regard, electronic system 500 may include the infusion pump 102 and/or a computing device within or connected to the infusion pump 102.

Electronic system 500 may include various types of computer readable media and interfaces for various other types of computer readable media. In the depicted example, electronic system 500 includes a bus 508, processing unit(s) 512, a system memory 504, a read-only memory (ROM) 510, a permanent storage device 502, input device interface(s) 514, output device interface(s) 506, and network interface(s) 516. In some implementations, electronic system 500 may include or be integrated with other computing devices or circuitry for operation of the various components and methods previously described.

Bus 508 collectively represents all system, peripheral, and chipset buses that communicatively connect the numerous internal devices of electronic system 500. For instance, bus 508 communicatively connects processing unit(s) 512 with ROM 510, system memory 504, and permanent storage device 502.

From these various memory units, processing unit(s) 512 retrieves instructions to execute and data to process, in order to execute the processes of the subject disclosure. The processing unit(s) can be a single processor or a multi-core processor in different implementations.

ROM 510 stores static data and instructions that are needed by processing unit(s) 512 and other modules of the electronic system. Permanent storage device 502, on the other hand, is a read-and-write memory device. This device is a non-volatile memory unit that stores instructions and data even when electronic system 500 is off. Some implementations of the subject disclosure use a mass-storage device (such as a magnetic or optical disk and its corresponding disk drive) as permanent storage device 502.

Other implementations use a removable storage device (such as a floppy disk, flash drive, and its corresponding disk drive) as permanent storage device 502. Like permanent storage device 502, system memory 504 is a read-and-write memory device. However, unlike storage device 502, system memory 504 is a volatile read-and-write memory, such as random access memory. System memory 504 stores some of the instructions and data that the processor needs at runtime. In some implementations, the processes of the subject disclosure are stored in system memory 504, permanent storage device 502, and/or ROM 510. From these various memory units, processing unit(s) 512 retrieves instructions to execute and data to process, in order to execute the processes of some implementations.

Bus 508 also connects to input and output device interfaces 514 and 506. Input device interface 514 enables the user to communicate information and select commands to the electronic system. Input devices used with input device interface 514 include, e.g., alphanumeric keyboards and pointing devices (also called "cursor control devices"). Output device interfaces 506 enables, e.g., the display of images generated by the electronic system 500. Output devices used with output device interface 506 include, e.g., printers and display devices, such as cathode ray tubes (CRT) or liquid crystal displays (LCD). Some implementations include devices such as a touchscreen that functions as both input and output devices.

Bus 508 also couples electronic system 500 to a network (not shown) through network interfaces 516. Network interfaces 516 may include, e.g., a wireless access point (e.g., Bluetooth or WiFi) or radio circuitry for connecting to a wireless access point. Network interfaces 516 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 500 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs).

In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software, depends upon the particular application and design constraints imposed on the overall system. The described functionality may be implemented in varying ways for each particular application.

## Claims

1. An infusion pump, comprising:
a housing (205) including a tubing receiving area (202);
an air-in-line sensor (210) including:
a first element (214) coupled to the housing behind the tubing receiving area (202); and
a second element (212) configured to be positioned over the tubing receiving area and biased by a constant force toward the first element (214);
a position sensor (228) coupled to the housing and configured to detect a position of the second element (212); and
a processor configured to:
receive confirmation that a fluid tubing (112) is installed in the tubing receiving area;
determine a distance between the first element (212) and the second element (214) based on the position of the second element detected by the position sensor (228);
set a baseline tubing thickness of the fluid tubing (112) according to the distance between the first element (214) and the second element (212);
configure an alarm threshold of the air-in-line sensor (210) according to the baseline tubing thickness; and
perform an infusion while operating the air-in-line sensor (210) according to the alarm threshold.

2. The infusion pump of claim 1, further comprising:
a door (204) movably coupled to the housing;
wherein the second element (212) is coupled to the door via a spring-loaded rod (222) that applies the constant force on the second element toward the first element (214) when the door is secured to the housing (205).

3. The infusion pump of claim 2, further comprising a magnet (226) coupled to the spring-loaded rod (222);
wherein the position sensor (228) is configured to detect the position of the second element (212) based on a position of the magnet (226).

4. The infusion pump of any one of claims 1-3, wherein:
the first element (214) is an ultrasonic wave receiver and the second element (212) is an ultrasonic wave transmitter; or
the first element (214) is an ultrasonic wave transmitter and the second element (212) is an ultrasonic wave receiver.

5. The infusion pump of any one of claims 1-4, wherein the baseline tubing thickness corresponds to the distance between the first element and the second element while the fluid tubing is installed in the tubing receiving area.

6. The infusion pump of any one of claims 1-5, wherein the constant force causes the second element to contact the fluid tubing without substantially obstructing a flow of fluid through the fluid tubing.

7. The infusion pump of any one of claims 1-6, wherein the constant force is selected from a range of 2-3 pounds per inch.

8. The infusion pump of any one of claims 1-7, wherein the processor is further configured to:
tune a range of readings of the air-in-line sensor using the baseline tubing thickness; and
configure the alarm threshold based on the tuned range of readings.

9. The infusion pump of any one of claims 1-8, wherein the processor is further configured to:
while performing the infusion, determine that the alarm threshold has been reached;
in response to determining that the alarm threshold has been reached, compare an output of the air-in-line sensor to an occlusion threshold; and
output an alarm based on the comparison of the output of the air-in-line sensor to the occlusion threshold.

10. The infusion pump of claim 9, wherein the processor is configured to:
output an occlusion alarm when the output of the air-in-line sensor is less than or equal to the occlusion threshold; and
output an air-in-line alarm when the output of the air-in-line sensor is greater than the occlusion threshold.

11. A method of operating the infusion pump of any one of claims 1-10, the method comprising:
at the processor of the infusion pump:
receiving confirmation that the fluid tubing is installed in the tubing receiving area of the infusion pump;
determining the distance between the first element and the second element of the air-in-line sensor of the infusion pump using the position sensor coupled to a housing of the infusion pump and configured to detect the position of the second element;
setting the baseline tubing thickness of the fluid tubing according to the distance between the first element and the second element;
configuring the alarm threshold of the air-in-line sensor according to the baseline tubing thickness.

12. The method of claim 11, further comprising, at the processor:
tuning a range of readings of the air-in-line sensor using the baseline tubing thickness; and
configuring the alarm threshold based on the tuned range of readings.

13. The method of any one of claims 11-12, further comprising, at the processor:
determining that the alarm threshold has been reached;
in response to determining that the alarm threshold has been reached, comparing an output of the air-in-line sensor to an occlusion threshold; and
outputting an alarm based on the comparison of the output of the air-in-line sensor to the occlusion threshold.

14. The method of claim 13, wherein:
outputting the alarm includes outputting an occlusion alarm when the output of the air-in-line sensor is less than or equal to the occlusion threshold; and
outputting the alarm includes outputting an air-in-line alarm when the output of the air-in-line sensor is greater than the occlusion threshold.

## Patentansprüche

1. Infusionspumpe, umfassend:
ein Gehäuse (205) mit einem Schlauchaufnahmebereich (202);
einen Luft-in-Leitung-Sensor (210), umfassend:
ein erstes Element (214), das mit dem Gehäuse hinter dem Schlauchaufnahmebereich (202) gekoppelt ist; und
ein zweites Element (212), das dazu ausgebildet ist, über dem Schlauchaufnahmebereich positioniert zu werden und mittels einer konstanten Kraft in Richtung des ersten Elements (214) vorgespannt zu sein;
einen Positionssensor (228), der mit dem Gehäuse gekoppelt ist und dazu ausgebildet ist, eine Position des zweiten Elements (212) zu detektieren; und
einen Prozessor, der dazu ausgebildet ist:
eine Bestätigung zu empfangen, dass ein Fluidschlauch (112) im Schlauchaufnahmebereich installiert ist;
einen Abstand zwischen dem ersten Element (212) und dem zweiten Element (214) auf der Grundlage der durch den Positionssensor (228) detektierten Position des zweiten Elements zu bestimmen;
eine Basis-Schlauchdicke des Fluidschlauchs (112) gemäß dem Abstand zwischen dem ersten Element (214) und dem zweiten Element (212) einzustellen;
einen Alarmschwellenwert des Luft-in-Leitung-Sensors (210) gemäß der Basis-Schlauchdicke zu konfigurieren; und
eine Infusion durchzuführen, während der Luft-in-Leitung-Sensor (210) gemäß dem Alarmschwellenwert betrieben wird.

2. Infusionspumpe nach Anspruch 1, ferner umfassend:
eine Tür (204), die beweglich mit dem Gehäuse gekoppelt ist;
wobei das zweite Element (212) über eine federbelastete Stange (222), die die konstante Kraft auf das zweite Element in Richtung des ersten Elements (214) ausübt, mit der Tür gekoppelt ist, wenn die Tür am Gehäuse (205) gesichert ist.

3. Infusionspumpe nach Anspruch 2, ferner umfassend einen Magneten (226), der mit der federbelasteten Stange (222) gekoppelt ist;
wobei der Positionssensor (228) dazu ausgebildet ist, die Position des zweiten Elements (212) auf der Grundlage einer Position des Magneten (226) zu detektieren.

4. Infusionspumpe nach einem der Ansprüche 1-3, wobei:
das erste Element (214) ein Ultraschallwellenempfänger und das zweite Element (212) ein Ultraschallwellensender ist; oder
das erste Element (214) ein Ultraschallwellensender und das zweite Element (212) ein Ultraschallwellenempfänger ist.

5. Infusionspumpe nach einem der Ansprüche 1-4, wobei die Basis-Schlauchdicke dem Abstand zwischen dem ersten Element und dem zweiten Element entspricht, während der Fluidschlauch im Schlauchaufnahmebereich installiert ist.

6. Infusionspumpe nach einem der Ansprüche 1-5, wobei die konstante Kraft bewirkt, dass das zweite Element den Fluidschlauch kontaktiert, ohne einen Fluidstrom durch den Fluidschlauch wesentlich zu behindern.

7. Infusionspumpe nach einem der Ansprüche 1-6, wobei die konstante Kraft aus einem Bereich von 2 bis 3 Pfund pro Zoll ausgewählt ist.

8. Infusionspumpe nach einem der Ansprüche 1-7, wobei der Prozessor ferner dazu ausgebildet ist:
einen Bereich von Messwerten des Luft-in-Leitung-Sensors unter Verwendung der Basis-Schlauchdicke abzustimmen; und
den Alarmschwellenwert auf der Grundlage des abgestimmten Bereichs von Messwerten zu konfigurieren.

9. Infusionspumpe nach einem der Ansprüche 1-8, wobei der Prozessor ferner dazu ausgebildet ist:
während der Durchführung der Infusion zu bestimmen, dass der Alarmschwellenwert erreicht worden ist;
als Reaktion auf die Bestimmung, dass der Alarmschwellenwert erreicht worden ist, einen Ausgang des Luft-in-Leitung-Sensors mit einem Okklusionsschwellenwert zu vergleichen; und
einen Alarm auf der Grundlage des Vergleichs des Ausgangs des Luft-in-Leitung-Sensors mit dem Okklusionsschwellenwert auszugeben.

10. Infusionspumpe nach Anspruch 9, wobei der Prozessor dazu ausgebildet ist:
einen Okklusionsalarm auszugeben, wenn der Ausgang des Luft-in-Leitung-Sensors kleiner als oder gleich dem Okklusionsschwellenwert ist; und
einen Luft-in-Leitung-Alarm auszugeben, wenn der Ausgang des Luft-in-Leitung-Sensors größer als der Okklusionsschwellenwert ist.

11. Verfahren zum Betreiben der Infusionspumpe nach einem der Ansprüche 1-10, wobei das Verfahren umfasst:
am Prozessor der Infusionspumpe:
eine Bestätigung zu empfangen, dass der Fluidschlauch im Schlauchaufnahmebereich der Infusionspumpe installiert ist;
den Abstand zwischen dem ersten Element und dem zweiten Element des Luft-in-Leitung-Sensors der Infusionspumpe unter Verwendung des Positionssensors zu bestimmen, der mit einem Gehäuse der Infusionspumpe gekoppelt ist und dazu ausgebildet ist, die Position des zweiten Elements zu detektieren;
die Basis-Schlauchdicke des Fluidschlauchs gemäß dem Abstand zwischen dem ersten Element und dem zweiten Element einzustellen;
den Alarmschwellenwert des Luft-in-Leitung-Sensors gemäß der Basis-Schlauchdicke zu konfigurieren.

12. Verfahren nach Anspruch 11, ferner umfassend, am Prozessor:
einen Bereich von Messwerten des Luft-in-Leitung-Sensors unter Verwendung der Basis-Schlauchdicke abzustimmen; und
den Alarmschwellenwert auf der Grundlage des abgestimmten Bereichs von Messwerten zu konfigurieren.

13. Verfahren nach einem der Ansprüche 11-12, ferner umfassend, am Prozessor:
zu bestimmen, dass der Alarmschwellenwert erreicht worden ist;
als Reaktion auf die Bestimmung, dass der Alarmschwellenwert erreicht worden ist, einen Ausgang des Luft-in-Leitung-Sensors mit einem Okklusionsschwellenwert zu vergleichen; und
einen Alarm auf der Grundlage des Vergleichs des Ausgangs des Luft-in-Leitung-Sensors mit dem Okklusionsschwellenwert auszugeben.

14. Verfahren nach Anspruch 13, wobei:
das Ausgeben des Alarms das Ausgeben eines Okklusionsalarms umfasst, wenn der Ausgang des Luft-in-Leitung-Sensors kleiner als oder gleich dem Okklusionsschwellenwert ist; und
das Ausgeben des Alarms das Ausgeben eines Luft-in-Leitung-Alarms umfasst, wenn der Ausgang des Luft-in-Leitung-Sensors größer als der Okklusionsschwellenwert ist.

## Revendications

1. Pompe à perfusion, comprenant :
un boîtier (205) comprenant une zone de réception de tubulure (202) ;
un capteur d'air en ligne (210) comprenant :
un premier élément (214) couplé au boîtier derrière la zone de réception de tubulure (202) ; et
un second élément (212) agencé pour être positionné au-dessus de la zone de réception de tubulure et être sollicité par une force constante vers le premier élément (214) ;
un capteur de position (228) couplé au boîtier et agencé pour détecter une position du second élément (212) ; et
un processeur agencé pour :
recevoir une confirmation selon laquelle une tubulure de fluide (112) est installée dans la zone de réception de tubulure ;
déterminer une distance entre le premier élément (212) et le second élément (214) sur la base de la position du second élément détectée par le capteur de position (228) ;
définir une épaisseur de tubulure de référence de la tubulure de fluide (112) en fonction de la distance entre le premier élément (214) et le second élément (212) ;
configurer un seuil d'alarme du capteur d'air en ligne (210) en fonction de l'épaisseur de tubulure de référence ; et
effectuer une perfusion tout en faisant fonctionner le capteur d'air en ligne (210) conformément au seuil d'alarme.

2. Pompe à perfusion selon la revendication 1, comprenant en outre :
une porte (204) couplée de manière mobile au boîtier ;
dans laquelle le second élément (212) est couplé à la porte via une tige à ressort (222) qui applique la force constante sur le second élément vers le premier élément (214) lorsque la porte est fixée au boîtier (205).

3. Pompe à perfusion selon la revendication 2, comprenant en outre un aimant (226) couplé à la tige à ressort (222) ;
dans laquelle le capteur de position (228) est agencé pour détecter la position du second élément (212) sur la base d'une position de l'aimant (226).

4. Pompe à perfusion selon l'une quelconque des revendications 1-3, dans laquelle :
le premier élément (214) est un récepteur d'ondes ultrasonores et le second élément (212) est un émetteur d'ondes ultrasonores ; ou
le premier élément (214) est un émetteur d'ondes ultrasonores et le second élément (212) est un récepteur d'ondes ultrasonores.

5. Pompe à perfusion selon l'une quelconque des revendications 1-4, dans laquelle l'épaisseur de tubulure de référence correspond à la distance entre le premier élément et le second élément tandis que la tubulure de fluide est installée dans la zone de réception de tubulure.

6. Pompe à perfusion selon l'une quelconque des revendications 1-5, dans laquelle la force constante amène le second élément à être en contact avec la tubulure de fluide sans obstruer de manière substantielle un écoulement de fluide à travers la tubulure de fluide.

7. Pompe à perfusion selon l'une quelconque des revendications 1-6, dans laquelle la force constante est sélectionnée dans une plage de 2 à 3 livres par pouce.

8. Pompe à perfusion selon l'une quelconque des revendications 1-7, dans laquelle le processeur est en outre agencé pour :
ajuster une plage de lectures du capteur d'air en ligne en utilisant l'épaisseur de tubulure de référence ; et
configurer le seuil d'alarme sur la base de la plage de lectures ajustée.

9. Pompe à perfusion selon l'une quelconque des revendications 1-8, dans laquelle le processeur est en outre agencé pour :
pendant l'exécution de la perfusion, déterminer que le seuil d'alarme a été atteint ;
en réponse à la détermination selon laquelle le seuil d'alarme a été atteint, comparer une sortie du capteur d'air en ligne à un seuil d'occlusion ; et
émettre une alarme sur la base de la comparaison de la sortie du capteur d'air en ligne au seuil d'occlusion.

10. Pompe à perfusion selon la revendication 9, dans laquelle le processeur est agencé pour :
émettre une alarme d'occlusion lorsque la sortie du capteur d'air en ligne est inférieure ou égale au seuil d'occlusion ; et
émettre une alarme d'air en ligne lorsque la sortie du capteur d'air en ligne est supérieure au seuil d'occlusion.

11. Procédé de fonctionnement de la pompe à perfusion selon l'une quelconque des revendications 1-10, le procédé comprenant :
au niveau du processeur de la pompe à perfusion :
recevoir une confirmation selon laquelle la tubulure de fluide est installée dans la zone de réception de tubulure de la pompe à perfusion ;
déterminer la distance entre le premier élément et le second élément du capteur d'air en ligne de la pompe à perfusion en utilisant le capteur de position couplé à un boîtier de la pompe à perfusion et agencé pour détecter la position du second élément ;
définir l'épaisseur de tubulure de référence de la tubulure de fluide en fonction de la distance entre le premier élément et le second élément ;
configurer le seuil d'alarme du capteur d'air en ligne en fonction de l'épaisseur de tubulure de référence.

12. Procédé selon la revendication 11, comprenant en outre, au niveau du processeur :
ajuster une plage de lectures du capteur d'air en ligne en utilisant l'épaisseur de tubulure de référence ; et
configurer le seuil d'alarme sur la base de la plage de lectures ajustée.

13. Procédé selon l'une quelconque des revendications 11-12, comprenant en outre, au niveau du processeur :
déterminer que le seuil d'alarme a été atteint ;
en réponse à la détermination selon laquelle le seuil d'alarme a été atteint, comparer une sortie du capteur d'air en ligne à un seuil d'occlusion ; et
émettre une alarme sur la base de la comparaison de la sortie du capteur d'air en ligne au seuil d'occlusion.

14. Procédé selon la revendication 13, dans lequel :
l'émission de l'alarme comprend l'émission d'une alarme d'occlusion lorsque la sortie du capteur d'air en ligne est inférieure ou égale au seuil d'occlusion ; et
l'émission de l'alarme comprend l'émission d'une alarme d'air en ligne lorsque la sortie du capteur d'air en ligne est supérieure au seuil d'occlusion.
